# EUROPEAN PATENT APPLICATION

(11) **EP 3 795 124 A1**
(43) Date of publication of application: **24.03.2021**
(21) Application number: 19803981.0
(22) Date of filing: 13.05.2019
(51) Int. Cl.: A61F 7/03

(54) **HEAT GENERATOR**

(30) Priority: 17.05.2018 JP 2018095369
(71) Applicant: Kobayashi Pharmaceutical Co., Ltd., Chuo-ku Osaka-shi, Osaka 541-0045 (JP)
(72) Inventor: MARUYAMA Tetsuya, Osaka-shi, Osaka 532-0033 (JP)
(74) Representative: Vossius & Partner Patentanwälte Rechtsanwälte mbB
(86) International application number: PCT/JP2019/018995
(87) International publication number: WO 2019/221079

(57) **Abstract**

A heat generator (1) comprises a heat-generating composition (2) that generates heat upon contact with air, a storage bag (3) that can store the heat-generating composition (2) in a storage part (32) inside thereof, and an adhesive layer (4) provided on one surface of the storage bag (3). The storage bag (3) has, in plan view, an outer shape having a constricted part (36) recessed inward between two convexly protruding first apex (33) and second apex (34); and the storage bag (3) is provided with a dividing part (7) that divides the storage part (32) and allows the storage bag (3) to be folded between the first apex (33) and the second apex (34).

## Description

### Technical Field

The present invention relates to a heat generator that is effective in alleviating pain caused by stiff shoulders.

### Background Art

Therapy for relieving pain, such as stiff shoulders, in various parts of the body includes hyperthermia therapy that warms the affected area. In hyperthermia therapy, disposable body warmers have conventionally been used to apply hyperthermic stimulation to the affected area. Such disposable body warmers use the heat generated by a heat-generating composition containing iron powder and an oxidation accelerator stored in a bag, as shown in PTL 1, for example. The heat-generating composition is suitable for hyperthermia therapy because it is adjusted to a temperature of about 45°C, and when attached to the skin, it can maintain the skin temperature in the range of about 38 to 42°C for a predetermined period of time.

### Citation List

### Patent Literature

PTL 1: JPH05-208031A

### Summary of Invention

### Technical Problem

For the purpose of treating stiff shoulders, a disposable body warmer is attached to the range from both shoulders to the neck of a person; however, it is difficult for the user to attach the disposable body warmer to the desired region on the back of the body. Moreover, disposable body warmers generally have a rectangular shape in plan view, and easily peel off at the curved portion of the shoulder or neck due to poor fit; accordingly, the effect of hyperthermia therapy cannot be sufficiently exerted.

The present invention has been made focusing on the above problems. An object of the present invention is to provide a heat generator that can be easily and accurately attached to a predetermined region by the user for relieving pain of stiff shoulders, and that is well fitted to the predetermined region.

### Solution to Problem

As a result of extensive study to solve the above problems, the present inventor found that among the trapezius and supraspinatus, the region from the base of the neck to the scapula (scapular region) feels particularly painful due to stiff shoulders, and that a heat generator having a shape that can be easily and accurately attached to this region can effectively apply hyperthermic stimulation to the trapezius and supraspinatus for a long period of time. The present invention has been completed as a result of further research based on these findings. That is, the present invention provides heat generators of the following aspects.

The heat generator according to the present invention is used by attachment to a scapular region of a person, the heat generator comprising:
a heat-generating material,
a storage bag that can store the heat-generating material in a storage part inside thereof, and
an adhesive layer provided on one surface of the storage bag;
wherein the storage bag has, in plan view, an outer shape having a constricted part recessed inward between two convexly protruding first apex and second apex, and
the storage bag is provided with a dividing part that divides the storage part and allows the storage bag to be folded between the first apex and the second apex.

A preferable embodiment of the heat generator according to the present invention is characterized in that the storage bag has, in plan view, a substantially triangular outer shape having a convexly protruding third apex in a position facing the constricted part.

Further, a preferable embodiment of the heat generator according to the present invention is characterized in that the dividing part is provided in the storage bag so as to extend between the constricted part and the third apex.

Further, a preferable embodiment of the heat generator according to the present invention is characterized in that the storage bag has, in plan view, an outer shape in which the first apex, the second apex, the third apex, and the constricted part are rounded, and side edges sandwiched between the adjacent apexes and between the adjacent apexes and the constricted part are each convexly curved outward.

Further, a preferable embodiment of the heat generator according to the present invention is characterized in that a virtual straight line connecting the tip of the constricted part and the tip of the first apex, and a virtual straight line connecting the tip of the constricted part and the tip of the second apex form an angle of 40° or more and 170° or less.

Further, a preferable embodiment of the heat generator according to the present invention is characterized in that a virtual straight line connecting the tip of the third apex and the tip of the first apex, and a virtual straight line connecting the tip of the third apex and the tip of the second apex form an angle of 50° or more and 120° or less.

Further, a preferable embodiment of the heat generator according to the present invention is characterized in that the storage bag has an axisymmetric outer shape in plan view.

### Advantageous Effects of Invention

The heat generator according to the present invention can be easily and accurately attached to a scapular region by the user *per se,* and is well fitted to the scapular region so that it is less likely to peel off from the scapular region. Therefore, the heat generator of the present invention can effectively apply hyperthermic stimulation, for a long period of time, to the trapezius in the scapular region where stiff shoulders are particularly painful.

### Brief Description of Drawings

Fig. 1 is a plan view of the heat generator.
Fig. 2 shows the bottom of the heat generator.
Fig. 3 shows an A-A cross-sectional view of Fig. 1.
Fig. 4 is a view for explaining a method of attaching the heat generator to a scapular region of a person.
Fig. 5 is a view showing a state of the heat generators attached to the scapular regions of a person.
Fig. 6 is a graph that evaluates the hyperthermic effect of the heat generator on stiff shoulder symptoms, and the continuity of the hyperthermic effect.

### Description of Embodiments

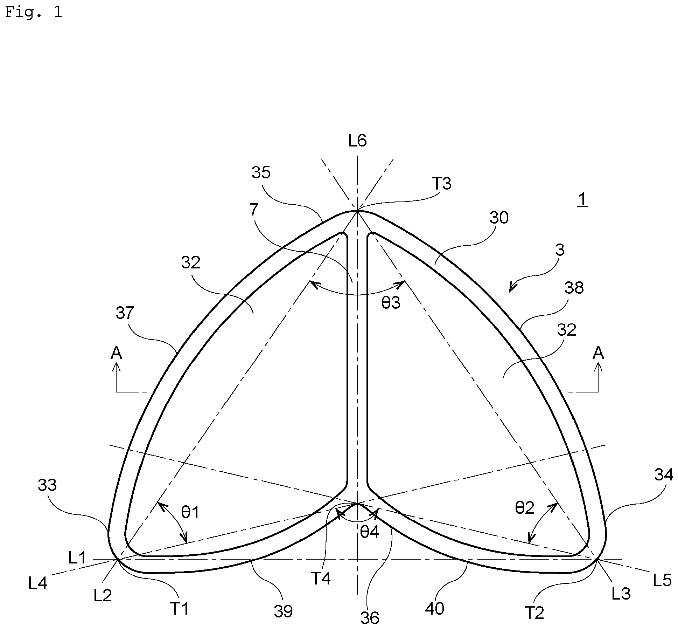

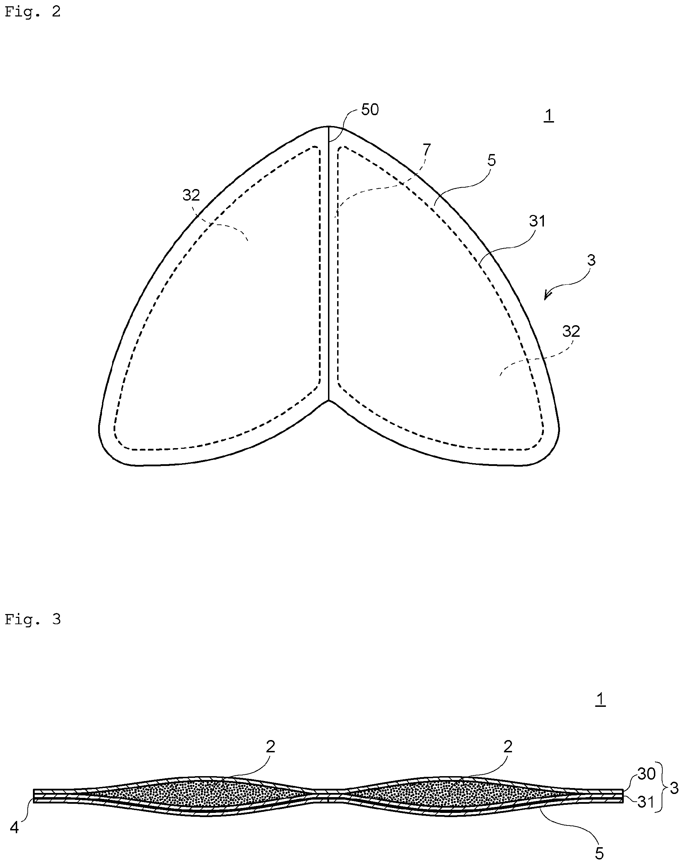

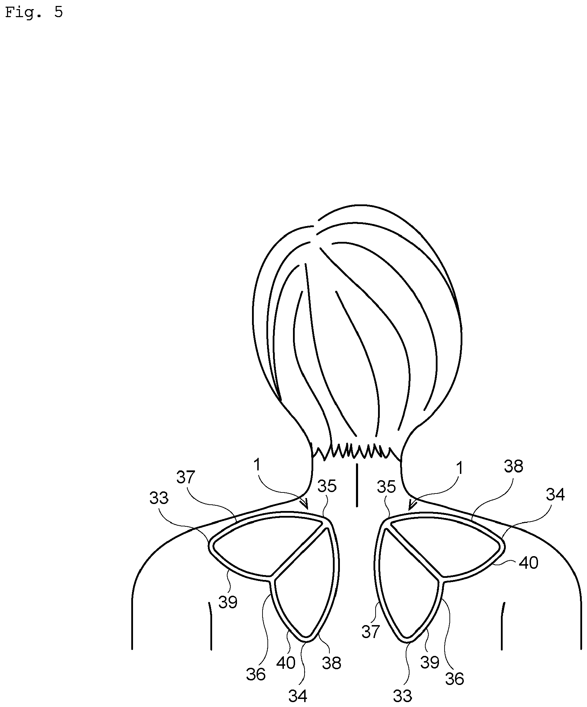

An embodiment of the heat generator according to the present invention is described below with reference to the drawings. Figs. 1 to 3 show the heat generator 1 of the present embodiment. The heat generator 1 is used to alleviate pain caused by stiff shoulders. As shown in Fig. 5, the heat generator 1 is attached to each of a pair of scapular regions of a person so as to cover the trapezius and supraspinatus for each scapular region, thereby effectively applying hyperthermic stimulation to the trapezius and supraspinatus.

The heat generator 1 comprises a heat-generating material 2, a storage bag 3 storing the heat-generating material 2, an adhesive layer 4 provided on one surface of the storage bag 3, and a release sheet 5 covering the adhesive layer 4. When using the heat generator 1, the release sheet 5 is removed from the storage bag 3, and the storage bag 3 is attached to the user's skin via the exposed adhesive layer 4, thereby applying the heat generated by the heat-generating material 2 in the storage bag 3 to the affected area as hyperthermic stimulation. As the heat generator 1, for example, a sticking-type disposable body warmer can be preferably used. Conventional disposable body warmers generally have a rectangular shape in plan view; however, the heat generator 1 of the present embodiment is different in shape from conventional disposable body warmers. Although it is not shown, the heat generator 1 is enclosed in a sealed flat outer bag when not in use.

Preferable examples of the heat-generating material 2 include a heat-generating composition that generates heat upon contact with air. The heat-generating composition may be any composition as long as it generates heat upon contact with air. Usable examples include known compositions conventionally used for disposable body warmers and containing appropriate amounts of oxidizable metal, activated carbon, water-retaining agents (e.g., wood powder, vermiculite, diatomaceous earth, perlite, silica gel, alumina, and water-absorbing resin), metal salts (e.g., common salt), and water. The oxidizable metal is a metal that generates heat of oxidation reaction, and examples include one or more powders or fibers selected from iron, aluminum, zinc, manganese, magnesium, and calcium. Among them, iron powder is preferable in terms of handleability, safety, production cost, storability, and stability. Examples of the iron powder include one or two or more members selected from reduced iron powder and atomized iron powder. Since the components of the heat-generating composition are adjusted, the heat generator 1 is controlled so as to satisfy the desired heat generation conditions (maximum temperature, average temperature, heat generation duration, etc.) when used.

The heat-generating material 2 may be a material other than the material that generates heat upon contact with air. Usable examples include materials that generate heat upon irradiation with microwaves by a microwave oven or the like (e.g., ceramic powder such as ferrite, and azuki beans).

The storage bag 3 comprises two front and back sheet materials 30 and 31 of the same shape. The two sheet materials 30 and 31 are superimposed, and the outer peripheral edges are joined over the entire circumference using a known adhesive or by heat fusion (heat-sealing), thereby forming a bag shape having a storage part 32 that can store the heat-generating material 2.

Of the two sheet materials 30 and 31, the first sheet material 30, which is not attached to the skin, is air-permeable. The first sheet material 30 may be a porous resin film or a resin film perforated with a needle as long as it is air-permeable, and may be a non-woven or woven fabric. Further, the first sheet material 30 may have a single-layer structure, such as a resin film or a non-woven fabric, or may have a multi-layer structure in which a resin film and a non-woven fabric are laminated. As the resin film, those that have been conventionally used as storage bags for disposable body warmers can be used. Examples include polyethylene, polypropylene, polyester, polyamide, polyvinyl alcohol, polyvinyl chloride, polyvinylidene chloride, polyurethane, polystyrene, ethylene-vinyl acetate copolymer, polycarbonate, rubber hydrochloride, and the like. Examples of the non-woven fabric include those containing artificial fibers, such as polypropylene, nylon, vinylon, polyester, polyethylene terephthalate, rayon, acetate, acrylic, polyethylene, and polyvinyl chloride; and those containing natural fibers, such as cotton, hemp, and silk. In addition to the non-woven fabrics, for example, paper can also be used.

The second sheet material 31, which is to be attached to the skin, may be air-permeable or air-impermeable. As with the first sheet material 30, the second sheet material 31 may be a resin film, a non-woven fabric, or a woven fabric, and may have a single-layer structure or a multi-layer structure.

The storage bag 3 has three convexly protruding first apex 33, second apex 34, and third apex 35 in plan view shape. The apexes 33 to 35 each protrude radially. Further, the storage bag 3 has a constricted part 36 that is recessed inward between the first apex 33 and the second apex 34. In the storage bag 3, the spaces between the adjacent apexes, and the spaces between the adjacent apexes and the constricted part 36, specifically the space between the first apex 33 and the third apex 35, the space between the second apex 34 and the third apex 35, the space between the first apex 33 and the constricted part 36, and the space between the second apex 34 and the constricted part 36, are connected by side edges 37, 38, 39, and 40, respectively, thereby forming an outer shape.

All of the side edges 37 to 40 may have a linear shape or a curved shape. Alternatively, some side edges may have a linear shape, and the other side edges may have a curved shape. The curved line may be a line that bends smoothly or a line that connects a plurality of straight lines at obtuse angles. Further, the curved line may be a convex curved line that bulges outward or a concave curved line that dents inward.

As described above, the storage bag 3 has, as a whole, a substantially triangular shape with the apexes 33 to 35 as vertices in plan view. The reason for referring to this shape as a substantially triangular shape is that since the constricted part 36 is present in a position facing the third apex 35, the shape is not a strictly triangular shape formed by virtual straight lines L1, L2, and L3, and the shape of each of the side edges 37 to 40 includes a curved shape. In Fig. 1, a virtual straight line connecting the first apex 33 and the second apex 34 is defined as L1, a virtual straight line connecting the first apex 33 and the third apex 35 is defined as L2, and a virtual straight line connecting the second apex 34 and the third apex 35 is defined as L3.

Each of the apexes 33 to 35 and the constricted part 36 may have a rounded shape or a shape with an angled tip in plan view. Specifically, the apexes 33 to 35 and the constricted part 36 are each formed by connecting two lines in a convex shape; for example, when straight lines are connected, when a straight line and a curved line (a convex curved line or a concave curved line) are connected, and when a convex curved line and a concave curved line are connected, the two lines are not connected smoothly, and thus the apexes 33, 34, and 35 and the constricted part 36 each have a shape with an angled tip. Further, when two convex curved lines are connected, if the two convex curved lines are not connected smoothly, that is, when the two convex curved lines are not connected smoothly so that the tangents at the end points have the same direction, the apexes 33 to 35 and the constricted part 36 each also have a shape with an angled tip. In this case, the positions of the angled tips of the apexes 33 to 35 and the constricted part 36 are the tips T1, T2, T3, and T4 of the apexes 33 to 35 and the constricted part 36.

On the other hand, when the two convex curved lines are connected smoothly, that is, when the two convex curved lines are connected smoothly so that the tangents at the end points have the same direction, the apexes 33 to 35 and the constricted part 36 each have a rounded shape. In this case, the tips T1, T2, and T3 of the apexes 33 to 35 are in the positions that protrude most outward in the radial direction in the apexes 33 to 35, and the tip T4 of the constricted part 36 is in the most inward position of the constricted part 36.

If the storage bag 3 has corners, the storage bag 3 will easily peel off; thus, in consideration of the difficulty of peeling off the storage bag 3, each of the apexes 33 to 35 and the constricted part 36 preferably has a rounded shape, rather than the above-mentioned shape with an angled tip. Similarly, it is preferable that each of the side edges 37 to 40 also has a convex curved shape.

The storage bag 3 has the constricted part 36 described above so that it can be attached to a person's scapular region with a good fit. The scapula is present in the scapular region. Since the scapular spine that extends left and right while inclining downward toward the inside in the upper part of the back, and the inner edge that extends vertically while inclining outward and downward in a position near the spine protrude on the skin side, the surface of the scapular region is uneven. In order to fit the storage bag 3 well to the protrusions of the scapular spine and inner edge of the scapula, the constricted part 36 is provided between the first apex 33 and the second apex 34 of the storage bag 3. Due to the constricted part 36, the side edges 39 and 40 sandwiched between the first apex 33 and the second apex 34 of the storage bag 3 form a substantially V-shape, whereby the side edges 39 and 40 are folded along the V-shape formed by the scapular spine and inner edge of the scapula, and closely adhere to the scapular region without floating from the surface thereof. Therefore, the storage bag 3 can be attached to the scapular region with a good fit, and the storage bag 3 is less likely to peel off from the scapular region.

Although the degree of constriction between the side edges 39 and 40 is not particularly limited, it is preferable to set the degree of constriction so that the side edges 39 and 40 are folded along the V-shape formed by the scapular spine and inner edge of the scapula, and so that a wide range of the trapezius and nearby supraspinatus on the scapular spine and inner edge of the scapula can be covered. For example, in Fig. 1, when a virtual straight line connecting the tip T1 of the first apex 33 and the tip T4 of the constricted part 36 is defined as L4, and a virtual straight line connecting the tip T2 of the second apex 34 and the tip T4 of the constricted part 36 is defined as L5, the angle θ4 formed by the virtual straight line L4 and the virtual straight line L5 is preferably 40° or more and 170° or less, and more preferably 100° or more and 160° or less.

Further, the storage bag 3 has a substantially triangular shape as described above so that it can cover a wide range of the trapezius and supraspinatus in the scapular region. The trapezius (including the supraspinatus) in the scapular region has a triangular shape as a whole from the base of the neck to the scapula, and the storage bag 3 is formed in a substantially triangular shape to match this triangular shape of the trapezius etc. in the scapular region. The storage bag 3 is not particularly limited as long as it has a substantially triangular shape; however, in order to cover a wider range of the trapezius and supraspinatus in the scapular region, for example, in Fig. 1, the angle θ3 formed by the virtual straight line L2 and the virtual straight line L3 is preferably 50° or more and 120° or less, and more preferably 50° or more and 90° or less. Moreover, the angle θ1 formed by the virtual straight line L2 and the virtual straight line L4 is preferably 10° or more and 60° or less, and more preferably 20° or more and 45° or less. In addition, the angle θ2 formed by the virtual straight line L3 and the virtual straight line L5 is preferably 10° or more and 60° or less, and more preferably 20° or more and 45° or less.

The storage bag 3 is provided with a dividing part 7 that divides the storage part 32 into two regions by joining the two sheet materials 30 and 31 to each other using an adhesive or by heat fusion (heat-sealing) in a predetermined position between the first apex 33 and the second apex 34. The area of the storage bag 3 can be reduced by bending the storage bag 3 so that the first apex 33 and the second apex 34 are folded at this dividing part 7. Therefore, the outer bag for enclosing the storage bag 3 when not in use can be made compact.

The dividing part 7 is provided in the storage bag 3 so as to extend between the third apex 35 and the constricted part 36 in the present embodiment. The dividing part 7 does not necessarily extend between the third apex 35 and the constricted part 36, and may be displaced to the left or right. Further, the dividing part 7 may be provided in the storage bag 3 so as to extend obliquely.

The dividing part 7 is formed by joining the two sheet materials 30 and 31 of the storage bag 3 using a known adhesive or by heat fusion (heat-sealing). The dividing part 7 may be formed by stitching the two sheet materials 30 and 31 of the storage bag 3.

The storage bag 3 has an outer shape axisymmetric to a virtual straight line L6 (center line in this embodiment) in plan view. As a result, one type of the heat generator 1 can be applied to both left and right scapular regions without distinction between left and right.

The adhesive layer 4 is provided on the surface of the air-impermeable second sheet material 31. The adhesive layer 4 is formed by applying an acrylic adhesive, a urethane adhesive, a silicon adhesive, a rubber adhesive, a pine resin, or the like that has an adhesive effect on the skin, to the surface of the second sheet material 31. Regarding the coating amount of the adhesive, it is preferably applied so that the adhesive layer 4 has a thickness of about 10 µm to 300 µm.

The release sheet 5 covers the adhesive layer 4 until the heat generator 1 is used. The release sheet 5 can be formed, for example, by laminating paper, polyethylene terephthalate, polyethylene, etc., alone or in combination, and subjecting the surface thereof on the side covering the adhesive layer 4 to silicon processing. The release sheet 5 is formed in the same shape as the second sheet material 31 so as to cover the entire surface of the second sheet material 31. The release sheet 5 is provided with a notch that divides the release sheet 5 into two parts. The release sheet 5 can thereby be easily removed separately from the storage bag 3.

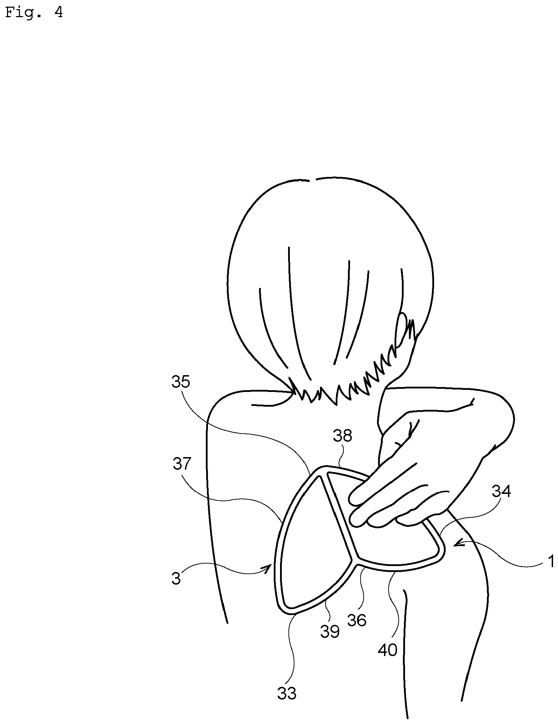

The heat generator 1 of the present embodiment described above is attached to a person's scapular region by the following procedure. First, the release sheet 5 is removed from the storage bag 3. Next, as shown in Fig. 4, the second apex 34 of the storage bag 3 is held by the left hand, and the left hand is turned toward the back of the person so that the third apex 35 faces inward with respect to the scapular region. Fig. 4 shows an example of attaching the heat generator 1 to the right scapular region. Then, the second apex 34 is attached to the scapular region so that the side edge 40 between the second apex 34 and the constricted part 36 of the storage bag 3 follows the scapular spine of the right scapula in the lower position of the scapular spine. At this time, the storage bag 3 can be folded between the first apex 33 and the second apex 34 at the dividing part 7. When the second apex 34 is attached to the scapular region, the first apex 33 side of the storage bag 3, which is initially folded on the second apex 34 side, is rotated around the dividing part 7 due to the moment when the second apex 34 is attached to the scapular region, and the first apex 33 side of the storage bag 3 is also attached to the scapular region. Thus, when the second apex 34 of the storage bag 3 is appropriately positioned with respect to the scapular region, the first apex 33 is attached to the scapular region so that the side edge 39 between the first apex 33 and the constricted part 36 of the storage bag 3 follows the inner edge of the scapula in the inner position of the inner edge, as shown in Fig. 5. Further, the third apex 35 is attached to the scapular region so that it faces the base of the neck. As a result, the heat generator 1 covers a wide range of the trapezius and supraspinatus from the base of the neck to the scapula.

Although it is not shown, in order to attach the heat generator 1 to the left scapular region, the first apex 33 of the storage bag 3 is held by the right hand, and the right hand is turned toward the back of the person so that the third apex 35 faces inward with respect to the scapular region. Then, the first apex 33 is attached to the scapular region so that the side edge 39 between the first apex 33 and the constricted part 36 of the storage bag 3 follows the scapular spine of the left scapula in the lower position of the scapular spine. As a result, as with the right scapular region, the second apex 34 side of the storage bag 3 can also be attached to the scapular region with the momentum when the first apex 33 is attached to the scapular region.

The heat generator 1 of the present embodiment described above can cover a wide range of the trapezius and supraspinatus in the scapular region, and thus can effectively apply hyperthermic stimulation to the trapezius and supraspinatus in the scapular region where stiff shoulders are often painful.

Further, for the scapular region on the back of the body, the user can easily and accurately attach the storage bag 3 to the scapular region by positioning a part of the storage bag 3 with respect to the scapular region.

Moreover, the storage bag 3 is provided with the constricted part 36. Due to the constricted part 36, the side edges 39 and 40 sandwiched between the first apex 33 and the second apex 34 are folded along the V-shape formed by the scapular spine and inner edge of the scapula, and closely adhere to the scapular region without floating from the surface thereof. Accordingly, the storage bag 3 can be attached to the scapular region with a good fit, and the storage part 3 can be made difficult to peel off from the scapular region. Therefore, hyperthermic stimulation can be continuously applied to the trapezius and supraspinatus in the scapular region for a long period of time.

In addition, the apexes 33 to 35 and the constricted part 36 of the storage bag 3 have a rounded shape, the side edges 37 to 40 also have a curved shape, and the storage bag 3 has no corners. Therefore, the storage bag 3 can be made more difficult to peel off from the scapular region.

The embodiment of the present invention has been described above; however, the present invention is not limited to the above embodiment, and various modifications can be made without departing from the spirit of the present invention.

For example, in the heat generator 1 of the above embodiment, the storage bag 3 has an axisymmetric outer shape in plan view; however, the storage bag 3 does not necessarily have an axisymmetric shape.

Further, in the heat generator 1 of the above embodiment, the storage bag 3 has the convexly protruding third apex 35 in the position facing the constricted part 36; however, the storage bag 3 does not necessarily have the third apex 35. For example, the portion corresponding to the third apex 35 may be formed flat without protruding in a convex shape, or the first apex 33 and the second apex 34 may be connected by an arc-shaped side edge.

### Examples

The present invention is described below with reference to Examples; however, the present invention is not limited to the following Examples.

For 35 subjects (20 years old to under 60 years old) who were aware of having stiff shoulders, heat generators with the shape shown in Fig. 1 were attached to cover the trapezius and supraspinatus in the scapular regions according to Fig. 5, and the effect on the stiff shoulder symptoms and the continuity of the hyperthermic effect were evaluated.

The heat generators used in this Example (of a size fitting a 160 × 160 mm square) were produced by the following procedure.

First, the following components were used as a heat-generating composition.

### Heat-generating composition

- Iron powder (average particle size: about 50 µm)
- Activated carbon (average particle size: about 100 µm)
- Water
- Vermiculite (average particle size: about 500 µm)
- Water-absorbing polymer (acrylic acid polymer partial salt crosslinked product, average particle size: 250 µm)
- Salt

The components of the heat-generating composition were mixed to obtain a mixture. The mixing ratios of iron powder, activated carbon, water, vermiculite, water-absorbing resin, and salt are 45 wt%, 16 wt%, 31 wt%, 3 wt%, 3 wt%, and 2 wt%, respectively. The resulting mixture was stored and sealed in an air-permeable storage bag made of a porous film (trade name: Breathron, produced by Nitoms, Inc.) having an air-impermeable adhesive sheet (trade name: Nitotac, produced by Nitoms, Inc.) attached to one surface thereof, and a dividing part was formed in the center of the storage bag, thereby obtaining a heat generator. Thereafter, the heat generator was quickly stored in an air-impermeable outer bag for disposable body warmers. The heat generator was obtained in this manner.

The resulting heat generator was taken out of the outer bag and measured. As a result, the maximum exothermic temperature was 45°C or lower, and the duration was 8 hours (according to JIS S4100, 2007). The skin temperature when the heat generator was attached was 39°C to 42°C.

The stiff shoulder symptoms were evaluated as follows.

One heat generator was applied to the affected area (trapezius (including supraspinatus) in the scapular region) for 8 hours a day, and this was repeated for 7 successive days.

As an evaluation before attachment of the heat generator (pre-confirmation), the stiff shoulder condition of each subject was evaluated in advance using a visual analogue scale (VAS). On the 1st day of the test, the evaluation of the VAS was performed before the heat generator was attached, 4 hours after attachment, 8 hours after attachment, and 8 hours after removal (16 hours after attachment). On the 2nd day to the 7th day after attachment, the evaluation was performed before attachment and 8 hours after attachment. Further, the evaluation of the VAS was also performed on the 8th to 14th days after the attachment was stopped.

The VAS is a conventionally known method for evaluating the degree of pain, and is a visual scale that indicates the degree of current pain by showing a 10-cm long black straight line ("no pain" at the left end, and "maximum imaginable pain" at the right end) to subjects. In this test, "no stiff shoulder (stiffness, pain)" was regarded as 0, and "severest imaginable stiff shoulder (stiffness, pain)" was regarded as 10. The degree of stiff shoulder (severity of the symptoms) was evaluated by the distance (unit: mm) from the left end 0 on the line by writing on the line how much the degree of stiff shoulder could be expressed between 0 and 10. The shorter the distance, the lower degree of stiff shoulder.

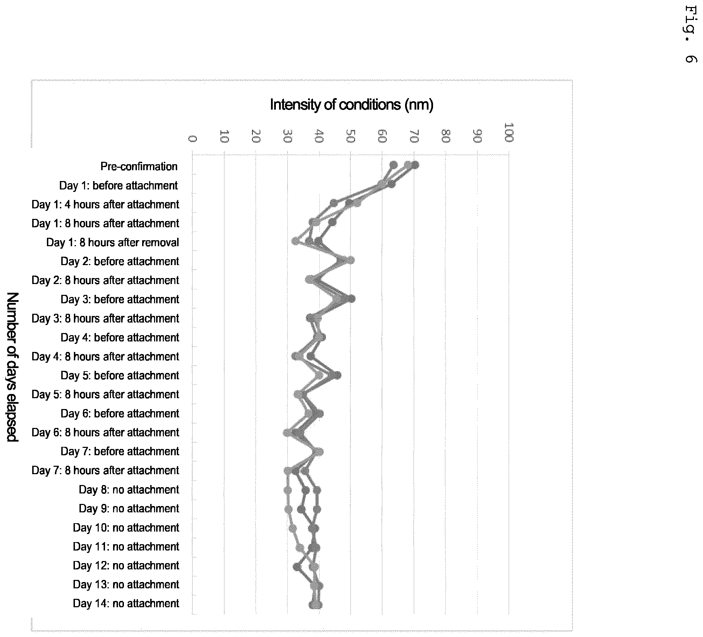

The results are shown in Fig. 6. As is clear from Fig. 6, although there was a pain of about 60 mm or more before the heat generator was attached (before the start of the test), the attachment of the heat generator improved the stiff shoulders during the attachment period. Further, after the 8th day from the start of attachment, the improvement of the stiff shoulders was maintained, even though the heat generator was not attached, compared to before the attachment of the heat generator (before the start of the test). This revealed that the heat generator was effective in alleviating pain. Moreover, when the subjects were asked to evaluate the degree of warmth, the result was such that a comfortable warmth could be obtained by attaching the heat generator to the trapezius and supraspinatus. In addition, the heat generator did not peel off from the affected area during attachment.

When the same test was performed for subjects with a degree of stiff shoulder of about 30 mm and subjects with a degree of stiff shoulder of 80 mm or more in the pre-confirmation by the VAS, their stiff shoulders were also improved by attaching the heat generator to the trapezius and supraspinatus, and a comfortable warmth was obtained.

### Reference Signs List

- 1.: Heat generator
- 2.: Heat-generating composition
- 3.: Storage bag
- 4.: Adhesive layer
- 5.: Release sheet
- 7.: Dividing part
- 32.: Storage part
- 33.: First apex
- 34.: Second apex
- 35.: Third apex
- 36.: Constricted part
- 37.: Side edge
- 38.: Side edge
- 39.: Side edge
- 40.: Side edge

## Claims

1. A heat generator that is used by attachment to a scapular region of a person, the heat generator comprising:
a heat-generating material,
a storage bag that can store the heat-generating material in a storage part inside thereof, and
an adhesive layer provided on one surface of the storage bag;
wherein the storage bag has, in plan view, an outer shape having a constricted part recessed inward between two convexly protruding first apex and second apex, and
the storage bag is provided with a dividing part that divides the storage part and allows the storage bag to be folded between the first apex and the second apex.

2. The heat generator according to claim 1, wherein the storage bag has, in plan view, a substantially triangular outer shape having a convexly protruding third apex in a position facing the constricted part.

3. The heat generator according to claim 2, wherein the dividing part is provided in the storage bag so as to extend between the constricted part and the third apex.

4. The heat generator according to claim 2 or 3, wherein the storage bag has, in plan view, an outer shape in which the first apex, the second apex, the third apex, and the constricted part are rounded, and side edges sandwiched between the adjacent apexes and between the adjacent apexes and the constricted part are each convexly curved outward.

5. The heat generator according to any one of claims 2 to 4, wherein a virtual straight line connecting the tip of the constricted part and the tip of the first apex, and a virtual straight line connecting the tip of the constricted part and the tip of the second apex form an angle of 40° or more and 170° or less.

6. The heat generator according to any one of claims 2 to 5, wherein a virtual straight line connecting the tip of the third apex and the tip of the first apex, and a virtual straight line connecting the tip of the third apex and the tip of the second apex form an angle of 50° or more and 120° or less.

7. The heat generator according to any one of claims 1 to 6, wherein the storage bag has an axisymmetric outer shape in plan view.
